Europäisches Patentamt

European Patent Office

Office européen des brevets

(19)

(11) Numéro de publication: **0 329 579 B1**

# FASCICULE DE BREVET EUROPEEN

(12)

(45) Date de publication de fascicule du brevet: **18.11.93**  (51) Int. Cl.5: **B01L 3/00**, C12M 1/16

(21) Numéro de dépôt: **89420054.2**

(22) Date de dépôt: **15.02.89**

(54) **Cupule d'analyse microbiologique ou similaire.**

(30) Priorité: **16.02.88 FR 8802180**

(43) Date de publication de la demande:
**23.08.89 Bulletin 89/34**

(45) Mention de la délivrance du brevet:
**18.11.93 Bulletin 93/46**

(84) Etats contractants désignés:
**AT BE CH DE ES FR GB GR IT LI LU NL SE**

(56) Documents cités:
**EP-A- 0 050 018**
**GB-A- 1 493 353**
**US-A- 3 363 503**
**US-A- 3 713 780**

(73) Titulaire: **BIO MERIEUX, Société anonyme**

**F-69280 Marcy l'Etoile(FR)**

(72) Inventeur: **Monget, Daniel**
**24 Résidence du Moulin**
**Saint-Sorlin-en-Bugey F-01150 Lagnieu(FR)**

(74) Mandataire: **Guerre, Dominique et al**
**Cabinet Germain et Maureau**
**20 Boulevard Eugène Deruelle**
**BP 3011**
**F-69392 Lyon Cédex 03 (FR)**

EP 0 329 579 B1

Rank Xerox (UK) Business Services
(3.10/3.6/3.3.1)

## Description

La présente invention concerne une cupule d'analyse microbiologique ou similaire du type comportant un fond plat, une paroi de section circulaire raccordée à ce fond plat et une extrémité ouverte.

Un tel type de cupule est employé dans certaines méthodes d'analyse, utilisées notamment à des fins de diagnostic rapide par identification, ou de détermination de sensibilité aux antibiotiques (antibiogramme) pour une souche bactérienne donnée. Ces méthodes sont déjà mises en oeuvre de façon standardisée dans certains laboratoires, à l'aide de plaques rectangulaires en matière synthétique et thermoformées, présentant une forme allongée et pourvues chacune, d'une ou de deux rangées de petites cupules tronconiques à fond plat constituant des tubes à essais miniaturisés. Chacune des cupules est initialement remplie soit d'un substrat de croissance déshydraté différent associé à un révélateur chimique approprié de consommation du substrat, soit d'un même antibiotique déshydraté à des concentrations différentes ou d'un antibiotique déshydraté différent.

Ces méthodes impliquent ensuite une opération d'ensemencement ou d'inoculation par introduction d'un volume donné de suspension aqueuse d'une souche microbienne dans chaque cupule. Suite à cette opération, les cupules sont mises à l'étuve pour une phase de croissance qui dure vingt-quatre heures.

Dans le cadre d'une identification bactérienne, la consommation du substrat propre à chaque cupule par la souche est visualisé par l'intermédiaire du révélateur chimique qui change la coloration du milieu de culture contenu dans la cupule. La lecture des colorations des différentes cupules se fait visuellement ou de préférence automatiquement par l'intermédiaire d'un photomètre équipé d'un passeur de filtres mesurant la lumière transmise par la suspension. L'utilisation par la souche inconnue des différents substrats des cupules de la plaque permet l'identification biochimique de la souche.

Dans le cadre de la détermination de la sensibilité d'une souche donnée aux antibiotiques, les résultats sont lus au moyen d'un photomètre mesurant par turbidimétrie le rapport de la lumière diffusée sur la lumière transmise par la suspension. La turbidité est maximale, lorsque la souche considérée est insensible à l'antibiotique testé et se développe normalement dans le milieu de culture.

Les photomètres utilisés dans les deux cas comportent une source lumineuse localisée au-dessus de la cupule concernée, émettant un faisceau lumineux incident perpendiculaire au fond plat de la cupule et trois diodes réceptrices dont l'une mesurant la lumière transmise est disposée directement au-dessous du fond plat, les deux autres diodes mesurant la lumière diffusée et décalées chacune d'environ 30° par rapport à celle mesurant la lumière transmise sont disposées en regard de la paroi de la cupule tronconique considérée.

Dans une première forme de réalisation actuellement connue, les cupules d'analyse présentent un fond plat raccordé à une paroi latérale leur conférant une configuration tronconique. En ce qui concerne leurs dimensions, elles possèdent un diamètre de base important et une hauteur faible. Le volume de suspension contenu dans chacune de celles-ci est d'environ 135 µl et la durée d'incubation en étuve nécessaire est de vingt-quatre heures.

Cette durée importante nuit à la rentabilité économique de ce type d'analyse microbiologique.

Compte-tenu de cela, il a été imaginé dans une seconde forme de réalisation connue de diminuer le diamètre de ces cupules tronconiques. Ainsi le trajet optique parcouru par le rayon lumineux incident du photomètre au-travers de la suspension bactérienne est, pour un même volume de 135 µl, plus long, ce qui a pour effet d'augmenter la sensibilité du photomètre de lecture et donc de permettre des mesures optiques fiables sur des suspensions bactériennes à faible durée de croissance, plus précisément quatre heures, pour lesquelles la révélation des réactions considérées n'est encore que naissante.

Il est à noter que le volume de suspension bactérienne contenue dans chacune de ces cupules reste cependant important, de l'ordre de 135 µl. Or, les galeries d'identification de souches et d'antibiogramme peuvent nécessiter jusqu'à trente-deux cupules, ce qui équivaut à un volume important de suspension bactérienne (six millilitres environ avec le volume mort) et donc à un nombre élevé de colonies nécessaires pour obtenir la suspension.

On sait, par ailleurs, que, lors d'une analyse microbiologique, le nombre de colonies bactériennes isolées à prélever doit être le plus faible possible. Ceci est dû, d'une part, au nombre limité de colonies disponibles pour un germe donné et, d'autre part, au risque de mélange bactérien au cours du prélèvement. Néanmoins, le taux de germes doit être suffisant pour obtenir une opacité standard des suspensions, indispensable pour garantir des résultats précis à la lecture.

Il est clair que des volumes importants de suspension bactérienne par cupule sont tout à fait incompatibles avec les contraintes de la pratique.

Compte-tenu de cela, il était donc nécessaire de mettre au point une cupule d'analyse contenant un faible volume de suspension bactérienne, et offrant un trajet optique relativement long au rayon lumineux incident du photomètre, afin de réduire la durée de la phase d'incubation à quatre heures

Le problème technique alors rencontré fut lié aux phénomènes de tension de surface de la suspension. En effet, un volume très faible de suspension introduit dans une cupule se présente sous la forme d'une gouttelette dont la cohésion est importante et qui, par conséquent, se moule difficilement à la forme définie par la paroi et le fond des cupules tronconiques connues. Ceci a pour conséquence que la gouttelette peut se fixer dans différentes positions sur la paroi de la cupule, du fait de la mouillabilité faible de celle-ci.

Son ménisque ne se trouve donc pratiquement jamais sensiblement compris dans un plan parallèle au fond de la cupule. De plus, même dans l'hypothèse la plus favorable de positionnement du ménisque, celui-ci est instable et évolue constamment d'une forme concave à une forme convexe ou inversement.

Ainsi, les résultats obtenus lors de la lecture colorimétrique ou turbidimétrique au-travers de telles cupules sont imprécis et faux. De plus, la reproductibilité des résultats obtenus n'est absolument pas assurée. A cela s'ajoute encore le fait que la variabilité de la forme du ménisque d'une cupule à une autre risque de fausser le résultat global de l'analyse.

Face à ces problèmes, la Demanderesse a engagé des recherches approfondies et de nombreux essais qui ont permis d'aboutir à une cupule tout à fait originale dans sa forme, permettant de remédier aux inconvénients et aux lacunes précités, capable de garantir des mesures photométriques fiables et reproductibles en vue d'analyses microbiologiques correctes, et ne nécessitant qu'un faible volume de suspension bactérienne et une courte durée d'incubation.

A cet effet, la cupule d'analyse microbiologique, objet de l'invention, est caractérisée en ce que ladite cupule est spécifiquement adaptée pour une identification biochimique d'un micro-organisme par détection optique, et à cette fin est préalablement remplie avec un milieu de culture, et en ce que la paroi est composée d'au moins trois zones superposées $Z_1$, $Z_2$, $Z_3$, ayant des angles d'inclinaison de la paroi respectivement différents, à savoir au moins une première zone inférieure tronconique $Z_1$ comportant le fond plat, et une deuxième zone, la bordure supérieure de la première zone étant reliée à la bordure inférieure de la deuxième zone par un bord annulaire compris dans un plan parallèle au fond plat, et une troisième zone tronconique $Z_3$ s'étend de la deuxième zone $Z_2$ à l'extrémité ouverte, avec la bordure inférieure de la troisième zone $Z_3$ constituant la bordure supérieure de la deuxième zone $Z_2$.

Conformément au document EP-A-0 050 018, et de manière spécifiquement adaptée à une analyse immunochimique ou immunologique, on a déjà décrit et proposé une cupule d'analyse, comprenant un fond plat, une paroi de section circulaire raccordée à ce fond plat jusqu'à une extrémité ouverte. Cette paroi présente à partir du fond plat une multiplicité, par exemple quatre zones tronconiques superposées, dont les diamètres de base respectifs sont croissants du fond plat vers l'extrémité ouverte de la cupule. Tous les angles d'inclinaison respectivement des multiples zones tronconiques sont sensiblement égaux. Et la bordure supérieure d'une zone donnée est reliée à la bordure inférieure de la zone suivante par un rebord annulaire incliné par rapport au fond plat de la cupule, à l'opposé de l'inclinaison commune des différentes zones tronconiques.

Une cupule pour analyse microbiologique selon la présente invention diffère d'une cupule pour analyse immunologique telle que précédemment décrite, d'une part par les caractéristiques de formes identifiées précédemment, et d'autre part, par son adaptation spécifique à une identification biochimique d'un micro-organisme, à savoir un remplissage préalable avec un milieu de culture.

Avec une cupule selon l'invention, le ménisque obtenu pour des faibles volumes de suspension bactérienne est relativement plat, c'est-à-dire sensiblement parallèle au fond de la cupule. Le bord de la cupule assure une bonne assise du ménisque et garantit ainsi sa stabilité dans le temps, autorisant une bonne précision et une bonne reproductibilité des mesures photométriques.

Selon un exemple non limitatif et intéressant, les hauteurs de la première zone tronconique $Z_1$, de la deuxième zone tronconique $Z_2$ et de la troisième zone tronconique $Z_3$ sont respectivement de l'ordre de 20 %, 50 % et 30 % de la hauteur totale de la cupule, tandis que le diamètre de base de la première zone tronconique $Z_1$, la différence entre le diamètre extérieur et le diamètre intérieur du bord annulaire, et le diamètre de base de la troisième zone tronconique $Z_3$ ont des longueurs respectivement égales à environ 43 %, 7 % et 69 % du diamètre supérieur de la troisième zone tronconique.

La troisième zone tronconique de la cupule évasée, facilite l'introduction de la suspension bactérienne et des réactifs, permet la mise en place d'huile ou de paraffine pour placer le milieu en anaérobie, et enfin favorise le centrage des cupules lors de leur gerbage les unes sur les autres en vue du stockage ou du conditionnement, ou lors de leur mise en place sur le support du photomètre. Par ailleurs, du fait de la concentration des couleurs sur une surface réduite délimitée par la deuxième zone tronconique $Z_2$, la lecture visuelle des réactions

tronconique $Z_3$, et inférieur à celui de la paroi dans la zone tronconique $Z_2$, et inférieur à celui de la paroi dans la zone tronconique $Z_3$.

Afin de faciliter le démoulage de la cupule lors de sa fabrication, de préférence, l'angle d'inclinaison de la paroi dans la zone tronconique $Z_1$ est supérieur à celui de la paroi dans la zone tronconi-que $Z_2$, et inférieur à celui de la paroi dans la zone tronconique $Z_3$.

La présente invention concerne également une plaque d'analyse microbiologique de forme allongée, rectangulaire, comprenant au moins une rangée de cupules alignées et espacées, chacune répondant à l'une quelconque des définitions précédentes.

De toute façon, l'invention sera mieux comprise à l'aide de la description qui suit, en référence au dessin schématique annexe représentant à titre d'exemples non limitatifs, deux formes d'exécution de cette cupule d'analyse microbiologique ou similaire :

Figure 1 est une vue en perspective de la cupule selon l'invention ;

Figure 2 est une vue en coupe diamétrale selon II-II de figure 1 ;

Figure 3 est une vue en perspective d'une plaque d'analyses microbiologiques ou similaires comportant des cupules selon l'invention.

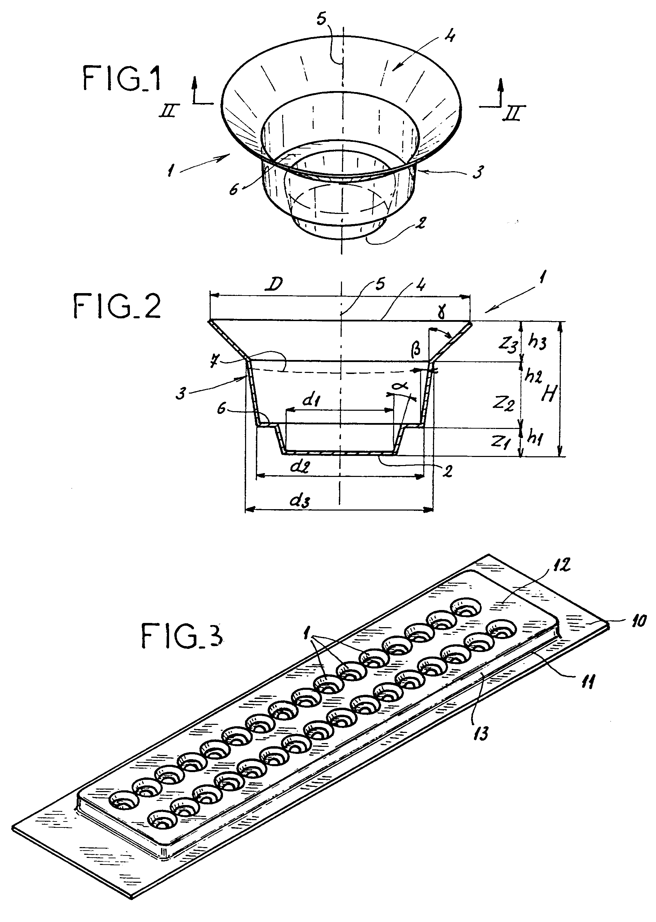

La cupule 1 représentée aux figures 1 et 2 est un récipient comportant un fond plat 2, une paroi 3 et une extrémité ouverte 4. Sa configuration est symétrique par rapport à son axe médian 5.

La paroi 3 possède une section transversale circulaire. Comme le montre la figure 2, elle présente, à partir du fond plat 2, trois zones tronconi-ques $Z_1$, $Z_2$, $Z_3$ de dimensions différentes. Leurs diamètres de base respectifs $d_1$, $d_2$ et $d_3$ sont croissants du fond plat 2 vers l'extrémité ouverte 4.

La première zone tronconique $Z_1$ de la paroi 3 est reliée à la deuxième zone tronconique $Z_2$ par l'intermédiaire d'un bord annulaire 6 plat compris dans un plan parallèle au fond plat 2. Le diamètre extérieur de ce bord annulaire 6 correspond au diamètre de base $d_2$ de la deuxième zone tronconi-que $Z_2$ de la paroi 3.

Cette deuxième zone tronconique $Z_2$ est raccor-dée à la troisième zone tronconique $Z_3$ évasée qui la prolonge jusqu'à l'extrémité ouverte 4 de la cupule 1. Le diamètre de base $d_3$ de cette troisiè-me zone tronconique $Z_3$ correspond au diamètre supérieur de cette deuxième zone tronconique $Z_2$.

La paroi 3 au niveau de la première zone tronconique $Z_1$ forme un angle $\alpha$ avec un plan perpendiculaire au fond plat 2 d'une valeur supérieure à celle d'un angle $\beta$ existant entre la paroi 3 au niveau de la deuxième zone tronconique $Z_2$ et un plan vertical perpendiculaire au bord annulaire 6 et inférieur à celle d'un angle $\gamma$ formé entre un plan vertical perpendiculaire à celui comprenant le fond plat 2 et la paroi 3 dans la troisième zone

tronconique $Z_3$.

Le ménisque 7 de la suspension bactérienne représenté en pointillés sur la figure 2 est relativement plat et parallèle au fond plat 2 du fait du bord annulaire 6 qui assure son assise et du fait des inclinaisons différentes de la paroi 3 dans les zo-nes tronconiques $Z_1$, $Z_2$ et $Z_3$ par rapport à la verticale.

De façon exemplative et non limitative, les dimensions relatives des différentes zones tronconiques de la paroi 3 et de la cupule 1 ont été déterminées comme suit : la hauteur totale de la cupule 1 étant désignée par H, les hauteurs $h_1$, $h_2$, $h_3$ des zones tronconiques $Z_1$, $Z_2$, $Z_3$ correspon-dantes représentent respectivement environ 21 %, 48 % et 31 % de la hauteur totale H de la cupule 1.

Concernant les diamètres des différentes zo-nes $Z_1$, $Z_2$, $Z_3$, si on considère le diamètre D de la troisième zone tronconique $Z_3$, c'est-à-dire de l'extrémité ouverte 4, le diamètre de base $d_1$ de la première zone tronconique $Z_1$ a une longueur re-présentant environ 45 % de celle de D, la différence entre le diamètre extérieur $d_2$ et le diamètre intérieur du bord annulaire 6 a une longueur représentant environ 7 % de celle de D et enfin le diamètre de base $d_3$ de la troisième zone tronconique a une longueur représentant environ 69 % de celle de D.

De façon plus précise, une cupule selon l'invention peut présenter les dimensions suivantes :

$H \approx 5\ mm \pm 2\ mm$     $D \approx 9,5\ mm$
$h_1 \approx 1\ mm$     $d_1 \approx 4,1\ mm$
$h_2 \approx 2,5\ mm$     $d_2 \approx 6\ mm$
$h_3 \approx 1,5\ mm$     $d_3 \approx 6,6\ mm$

Le volume contenu par cupule peut, dans cet exemple, varier de 50 µl à 60 µl.

Pour des contenances par cupule différentes, les dimensions varieront selon les normes définies ci-dessus.

Il faut encore signaler que les cupules selon l'invention sont obtenues notamment par thermoformage d'un matériau synthétique.

En vue de l'utilisation de la cupule, il est prévu avantageusement une plaque-support de cupule, comme celle représentée à la figure 3. La plaque 10 présente une forme allongée rectangulaire. Elle est délimitée par des bords 11 formant partie d'appui. Ces bords 11 sont reliés au plan supérieur 12 de la plaque 10 par l'intermédiaire de parois 13 perpendiculaires à ceux-ci. Le plan supérieur 12 est parallèle au plan comprenant les bords 11 d'appui et est raccordé aux bords des extrémités ouvertes des cupules 1 selon l'invention, groupées en rangées, le nombre des cupules 1 représenté sur la figure 4 n'étant pas limitatif. Ces plaques-support 10 de cupules 1 d'analyse microbiologique sont classiquement obtenues par thermoformage d'un matériau synthétique approprié.

**Revendications**

1. Cupule (1) d'analyse formant tube à essai miniaturisé, comprenant un fond plat (2), une paroi (3) de section circulaire raccordée à ce fond plat jusqu'à une extrémité ouverte (4), ladite paroi présentant à partir du fond plat une pluralité de zones tronconiques superposées, ayant chacune une bordure supérieure et une bordure inférieure, dont les diamètres de base respectifs sont croissants du fond plat vers l'extrémité ouverte, **caractérisée en ce que** ladite cupule (1) est spécifiquement adaptée pour une identification biochimique d'un microorganisme par détection optique, et à cette fin est préalablement remplie avec un milieu de culture, et en ce que la paroi (3) est composée d'au moins trois zones superposées ($Z_1$, $Z_2$, $Z_3$), ayant des angles d'inclinaison de la paroi (3) respectivement différents, à savoir au moins une première zone inférieure tronconique ($Z_1$) comportant le fond plat (2), et une deuxième zone, la bordure supérieure de la première zone étant reliée à la bordure inférieure de la deuxième zone par un bord annulaire (11) compris dans un plan parallèle au fond plat (2), et une troisième zone tronconique ($Z_3$) s'étend de la deuxième zone ($Z_2$) à l'extrémité ouverte (4), avec la bordure inférieure de la troisième zone ($Z_3$) constituant la bordure supérieure de la deuxième zone ($Z_2$).

2. Cupule selon la revendication 1, caractérisée en ce que la hauteur de la première zone tronconique ($Z_1$) est inférieure à celle de la deuxième ($Z_2$) ou troisième zone ($Z_3$).

3. Cupule selon la revendication 1, caractérisée en ce que l'angle d'inclinaison ($\alpha$) de la paroi (3) dans la première zone tronconique ($Z_1$) est supérieur à l'angle d'inclinaison ($\beta$) de la paroi (3) dans la deuxième zone tronconique ($Z_3$), et inférieur à l'angle d'inclinaison ($\gamma$) de la paroi (3) dans la troisième zone tronconique ($Z_3$).

4. Plaque (10) d'analyse microbiologique, de forme allongée rectangulaire, comprenant au moins une rangée de cupules alignées et espacées (1), chacune conforme à l'une quelconque des revendications 1 à 3.

**Claims**

1. Analysis well (1) forming a miniature test tube having a flat bottom (2), a circular cross-section wall (3) connected to said flat bottom up to an open end (4), said wall having starting from the flat bottom a plurality of superposed frustoconical areas each having an upper edge and a lower edge whose respective base diameters increase from the flat bottom to the open end, **characterised in that** said well (1) is specifically adapted for biochemical identification of a microorganism by optical means and to this end is filled beforehand with a culture medium and in that the wall (3) comprises at least three superposed areas ($Z_1$, $Z_2$, $Z_3$) having respective different angles of inclination of the wall (3), namely at least a first frustoconical lower area ($Z_1$) comprising the flat bottom (2) and a second area, the upper edge of the first area being connected to the lower edge of the second area by an annular rim (11) contained in a plane parallel to the flat bottom (2), and a third frustoconical area ($Z_3$) extends from the second area ($Z_2$) to the open end (4) with the lower edge of the third area ($Z_3$) constituting the upper edge of the second area ($Z_2$).

2. Well according to claim 1 characterised in that the height of the first frustoconical area ($Z_1$) is less than that of the second area ($Z_2$) or the third area ($Z_3$).

3. Well according to claim 1 characterised in that the angle of inclination ($\alpha$) of the wall (3) in the first frustoconical area ($Z_1$) is greater than the angle of inclination ($\beta$) of the wall (3) in the second frustoconical area ($Z_2$) and less than the angle of inclination ($\gamma$) of the wall (3) in the third frustoconical area ($Z_3$).

4. Microbological analysis plate (10) of elongate rectangular shape comprising at least one row of aligned and spaced wells (1) each according to any one of claims 1 to 3.

**Patentansprüche**

1. Analysenschale (1) nach Art eines miniaturisierten Reagenzglases mit einem ebenen Boden (2), mit einer Wand (3) mit kreisförmigem Querschnitt, die sich von dem ebenen Boden bis zu einem offenen Ende (4) erstreckt, wobei die Wand, ausgehend vom ebenen Boden, eine Vielzahl übereinander angeordneter, kegelstumpfförmiger Zonen ausbildet, von denen jede eine obere Kante und eine untere Kante hat und deren jeweilige Basis-Durchmesser vom ebenen Boden zum offenen Ende hin anwachsen,

**dadurch gekennzeichnet,**

daß die Schale (1) spezifisch für die biochemische Identifikation eines Mikroorganismus durch optischen Nachweis angepaßt ist und zu diesem Zweck zunächst mit einem Kulturmilieu gefüllt wird, und daß die Wand (3) aus wenigstens drei übereinander angeordneten Zonen (Z1, Z2, Z3) zusammengesetzt ist, die jeweils unterschiedliche Neigungswinkel der Wand (3) haben, nämlich wenigstens eine erste, untere, kegelstumpfförmige Zone (Z1), die den ebenen Boden (2) enthält, eine zweite Zone, wobei der obere Rand der ersten Zone mit dem unteren Rand der zweiten Zone über eine ringförmige Kante (6) verbunden ist, die sich in einer Ebene parallel zum ebenen Boden (2) erstreckt, und eine dritte kegelstumpfförmige Zone (Z3), die sich ausgehend von der zweiten Zone (Z2) zum offenen Ende (4) erstreckt, wobei der untere Rand der dritten Zone (Z3) den oberen Rand der zweiten Zone (Z2) ausbildet.

2. Analysenschale nach Anspruch 1,
   **dadurch gekennzeichnet,**
   daß die Höhe der ersten kegelstumpfförmigen Zone (Z1) kleiner ist als diejenige der zweiten Zone (Z2) oder der dritten Zone (Z3).

3. Analysenschale nach Anspruch 1,
   **dadurch gekennzeichnet,**
   daß der Neigungswinkel ($\alpha$) der Wand (3) in der ersten kegelstumpfförmigen Zone (Z1) größer ist als der Neigungswinkel ($\beta$) der Wand (3) in der zweiten kegelstumpfförmigen Zone (Z2) und kleiner als der Neigungswinkel ($\gamma$) der Wand (3) in der dritten kegelstumpfförmigen Zone (Z3).

4. Platte (10) zur mikrobiologischen Analyse mit einer länglichen, rechteckigen Form, die wenigstens eine Reihe von zueinander ausgerichteten und voneinander getrennten schalen (1) aufweist, von denen jede einem der Patentansprüche 1 bis 3 entspricht.

FIG.1

FIG.2

FIG.3